# EUROPEAN PATENT APPLICATION

(11) **EP 4 491 713 A1**
(43) Date of publication of application: **15.01.2025**
(21) Application number: 24186424.8
(22) Date of filing: 04.07.2024
(51) Int. Cl.: C12N 5/077

(54) **CELL REPAIR-PROMOTING EXTRACELLULAR VESICLE, PRODUCTION METHOD, PREPARATION, AND USE THEREOF**

(30) Priority: 06.07.2023 US 202363512091 P
(71) Applicant: China Medical University, Taichung City 406040 (TW)
(72) Inventor: Chen, Yi-Wen, 404 Taichung City (TW); Shie, Ming-You, 404 Taichung City (TW); Lee, Jian-Jr, 404 Taichung City (TW); Cho, Der-Yang, 404 Taichung City (TW); Chen, Cheng-Yu, 404 Taichung City (TW); Lin, Yen-Hong, 404 Taichung City (TW)
(74) Representative: Cabinet Chaillot

(57) **Abstract**

A cell repair-promoting extracellular vesicle, formed by silicate-induced stem cell secretion, helps express anti-inflammatory, tissue regeneration, and cell repair indicator factors, accelerating cell growth in wound tissue and the wound repair process.

## Description

### Field of the Invention:

An extracellular vesicle, in particular an extracellular vesicle which promotes the repair of cells, a method for the production, a preparation, and a use thereof.

### Background of the Invention:

There are many potential factors that commonly lead to poor wound healing, ranging from inadequate wound cleaning/care to the patient's physical condition, such as advanced age, malnutrition, diabetes, autoimmune disease, cancer, or unhealthy habits such as smoking and drinking, all of which are closely linked to the rate at which wounds heal.

In addition to debridement, use of antibiotics and wound dressings, new treatments include topical application of human growth factors, negative pressure therapy, hyperbaric oxygen therapy, and biotechnological skin grafts. However, these are not only expensive, but their ability to accelerate wound healing is still limited. Therefore, the development of technologies to accelerate wound healing remains an urgent research goal in related fields.

### Summary of the Invention:

To develop a technology that promotes in accelerating wound healing, the present invention provides an extracellular vesicle that promotes in cell repair, specifically a silicate-induced extracellular vesicle formed by silicate-induced stem cell secretion.

The steps for producing the silicate-induced extracellular vesicles include:
adding 1 g of calcium silicate to 10 to 100 ml of a culture medium, soaking for at least 24 hours, then collecting the clarified solution and removing calcium elements to prepare a silicate culture medium;
culturing stem cells in the silicate culture medium; and
isolating silicate-induced extracellular vesicles secreted by the stem cells.

The culture medium is either a mesenchymal stem cell medium (MSCM) or a Dulbecco's modified Eagle's medium (DMEM).

The present invention also provides a cell repair-promoting preparation containing silicate-induced extracellular vesicles (CSEVs) formed by silicate-induced stem cell secretion.

It comprises a hydrogel used to carry these silicate-induced extracellular vesicles (CSEVs).

The proportion of these silicate-induced extracellular vesicles (CSEVs) in the hydrogel ranges from 10⁹-10¹⁴ particles/mL.

The silicate-induced extracellular vesicle (CSEV) provided by the present invention has a significantly increased surface potential, total protein content, and transmembrane protein content compared to an ordinary extracellular vesicle, which helps to increase the affinity of the silicate-induced extracellular vesicle (CSEV) for endocytosis by recipient cells. Through a cell and animal experiment, it has been confirmed that the silicate-induced extracellular vesicle (CSEV) can easily enter the cell by endocytosis and help express indicator factors for anti-inflammation, tissue regeneration and cell repair, as well as accelerate cell growth in wound tissue and wound repair process.

### Brief Description of the Drawings

FIG. 1 shows the change in calcium and silicon ion content in the silicate culture medium provided by the present invention;
FIG. 2 shows the cell viability test after stem cell culture provided by the present invention;
FIG. 3A shows the transmission electron microscope imaging of extracellular vesicles provided by the present invention;
FIG. 3B shows the nanoparticle tracking analysis of extracellular vesicles provided by the present invention;
FIG. 3C shows the membrane surface potential test of extracellular vesicles provided by the present invention;
FIG. 3D shows the protein expression level test of extracellular vesicles provided by the present invention;
FIGS. 3E to 3G show the analysis of transmembrane protein CD9, CD63, and CD81 expression ratios in extracellular vesicles provided by the present invention;
FIG. 3H shows the protein expression level imaging of extracellular vesicles provided by the present invention;
FIG. 4A shows the cell viability test of the diabetic cell model provided by the present invention;
FIG. 4B shows the intracellular reactive oxygen species test of the diabetic cell model provided by the present invention;
FIG. 4C shows the cell fluorescence staining of the diabetic cell model provided by the present invention;
FIG. 4D shows the cell fluorescence expression level of the diabetic cell model provided by the present invention;
FIG. 5A shows the cell viability test of the diabetic cell model after reaction with extracellular vesicles provided by the present invention;
FIG. 5B shows the intracellular reactive oxygen species test of the diabetic cell model after reaction with extracellular vesicles provided by the present invention;
FIG. 5C shows the wound healing assay imaging of the diabetic cell model after reaction with extracellular vesicles provided by the present invention;
FIG. 5D shows the cell migration ratio of the diabetic cell model after reaction with extracellular vesicles provided by the present invention;
FIG. 6 shows a schematic diagram of hydrogel-carried extracellular vesicles and cell culture provided by the present invention;
FIG. 7 shows the cell viability test of the diabetic cell model after culture with hydrogel-carried extracellular vesicles provided by the present invention;
FIG. 8 shows the expression level test of indicator factors in the diabetic cell model after culture provided by the present invention;
FIGS. 9A to 9C show the miRNA expression level analysis of the diabetic cell model after culture provided by the present invention; and
FIG. 10 shows the wound repair section staining of the diabetic animal model provided by the present invention.

### Detailed Description of the Invention:

It should be understood that the term "extracellular vesicle (EV)" as used in this document refers to a bilayer lipid vesicle secreted by cells into the extracellular space. The extracellular vesicle includes an exosome and a microvesicle, and as a new form of intercellular communication, it represents a new field of research in physiology, pathology, nanoscience, and other fields in recent years. The extracellular vesicle is a highly heterogeneous vesicular body secreted by cells, which contains proteins, mRNA/miRNA, DNA, lipids, and so on. It is classified based on its biogenesis, release pathway, size, content, and function, with purification method mainly distinguishing it by particle size.

It should be understood that the following combines the specification, drawings, and specific embodiments to illustrate the present invention, but the embodiments do not limit the present invention in any form. Unless otherwise indicated, the reagents, methods, and apparatus used in the present invention are conventional reagents, methods, and apparatus in the art.

The present invention provides a silicate-induced extracellular vesicle (CSEV) secreted by silicate-induced stem cells. Preferably, the stem cell is a human stem cell, which may optionally include, but is not limited to, a human adipose-derived stem cell (ADSC), a bone marrow-derived stem cell (BMSC), a dental pulp stem cell (DPSC), or a Wharton's jelly MSC (WJMSC), etc.

In this embodiment, the human adipose-derived stem cell (ADSC) is used as the source cell for silicate-induced extracellular vesicle secretion for the following explanation. By culturing the human stem cells in a silicate culture medium containing a silicate component, the human adipose-derived stem cells (ADSCs) are induced to secrete the extracellular vesicles. The extracellular vesicles secreted after induction by the silicate culture medium are further defined in this document as silicate-induced extracellular vesicles (CSEVs).

The culture method for these human Adipose-derived stem cells (ADSCs) is not limited, and any method that can culture these human Adipose-derived stem cells (ADSCs) and obtain the silicate-induced extracellular vesicles (CSEVs) is covered within the scope of the present invention.

The silicate refers to a silicic acid compound (SiₓO_{y}) composed of silicon and oxygen. The silicic acid compound further includes one or more metals or hydrogen. In this embodiment, the silicate is a calcium silicate.

The culture medium used for adding the silicate and culturing the human adipose-derived stem cells is not limited and can be a mesenchymal stem cell medium (MSCM, ScienCell Research Laboratories), a Dulbecco's modified Eagle's medium (DMEM), or other common cell culture media, all of which are covered within the scope of the present invention.

Furthermore, the steps for culturing the silicate-induced extracellular vesicles (CSEVs) include:
Step S1-1, Preparation of Silicate Culture Medium: Add 1 g of calcium silicate to 10-100 ml of the mesenchymal stem cell medium (MSCM), soak for at least 24 hours, and collect the clarified solution to prepare the silicate culture medium.

Wherein, the collection of the clarified solution can be achieved by filtration or centrifugation.

Wherein, during the soaking for at least 24 hours, a temperature environment or shaker can be provided to promote the reaction of the calcium silicate in the mesenchymal stem cell medium.

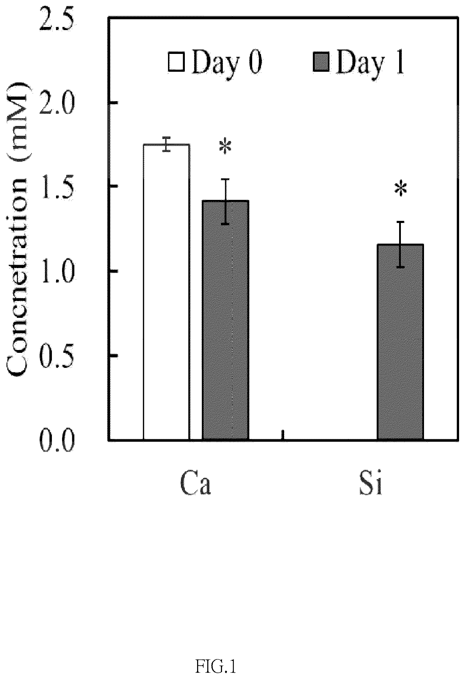

Reference is made to FIG. 1, which further analyzes the changes in calcium and silicon ion content in the mesenchymal stem cell medium when the calcium silicate is added using Inductively Coupled Plasma Mass Spectrometry (ICP-MS) technology. On day 0, there is a significant release of calcium ions due to the addition of the calcium silicate to the mesenchymal stem cell medium. After the calcium silicate reacts in the mesenchymal stem cell medium for 24 hours and is made into the silicate culture medium, the calcium and silicon ion content in the prepared silicate culture medium can be clearly measured, confirming the effectiveness of the calcium silicate reaction in the mesenchymal stem cell medium.

Step S1-2, Cultivation of human adipose-derived stem cells (ADSCs) in the silicate culture medium and incubation in a cell culture incubator at 37°C with 5% CO₂.

The human adipose-derived stem cells (ADSC) can be cultured in a T175 culture flask filled with the mesenchymal stem cell medium. The concentration of the silicate culture medium in the mesenchymal stem cell medium ranges from 5% to 50%.

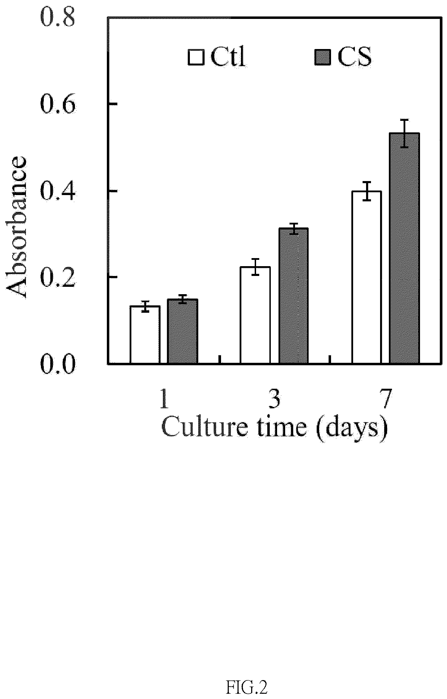

Reference is made to FIG. 2, which further defines the human adipose-derived stem cells (ADSCs) cultured in the silicate culture medium as a cell culture embodiment; and the cultivation of the human adipose-derived stem cells (ADSCs) in the mesenchymal stem cell medium serves as a cell culture comparative example. Through a cell viability test to examine cell proliferation effects, it can be seen that from the third day onwards, the number of the human adipose-derived stem cells (ADSCs) cultured in the silicate culture medium is significantly higher than in the cell culture comparative example, suggesting that the human adipose-derived stem cells (ADSCs) cultured in the silicate culture medium show a cell proliferation effect.

The cell viability test for cell proliferation effect can be achieved through methods such as MTT assay, PrestroBlue assay, or Alamar Blue assay, by detecting fluorescence or absorbance to analyze cell survival rate and proliferation effect. In this embodiment, the cell viability test is achieved using the PrestroBlue assay.

Step S1-3, Isolation of Silicate-induced Extracellular Vesicles (CSEVs): Collect the cultured human adipose-derived stem cells (ADSCs) together with the silicate culture medium, centrifuge at 1500 g for 5 minutes, and then collect the resulting first supernatant. Then filter the first supernatant through a 0.22-micron (µm) filter and place it in a concentration centrifuge tube (Amicon^{®} Ultra-15 Centrifugal Filter Unit, Merck) and centrifuge at 5000 g for 15 minutes. After which the silicate-induced extracellular vesicles (CSEVs) will remain in the resulting second supernatant. Then collect the second supernatant and use the principle of polymer precipitation to utilize the characteristic of high molecular weight polyethylene glycol to compete with free water molecules and bind to hydrophobic proteins and lipid molecules, the less soluble silicate-induced extracellular vesicles (CSEVs) are precipitated from the second supernatant. Finally, centrifuge at 1500 g for 30 to 40 minutes to remove the supernatant, and resuspend in a Phosphate Buffered Saline (PBS) to obtain the high-purity silicate-induced extracellular vesicles (CSEVs) after precipitation.

Experiment 1: Analysis of Silicate-induced Extracellular Vesicles (CSEVs) Characteristics.

The present invention further compares the collected silicate-induced extracellular vesicles (CSEVs) with the ordinary extracellular vesicles (EVs) obtained from the comparative example culture in terms of protein expression levels, membrane surface charge, and particle size to identify the appearance characteristics and various cell physiological data of the silicate-induced extracellular vesicles (CSEVs).

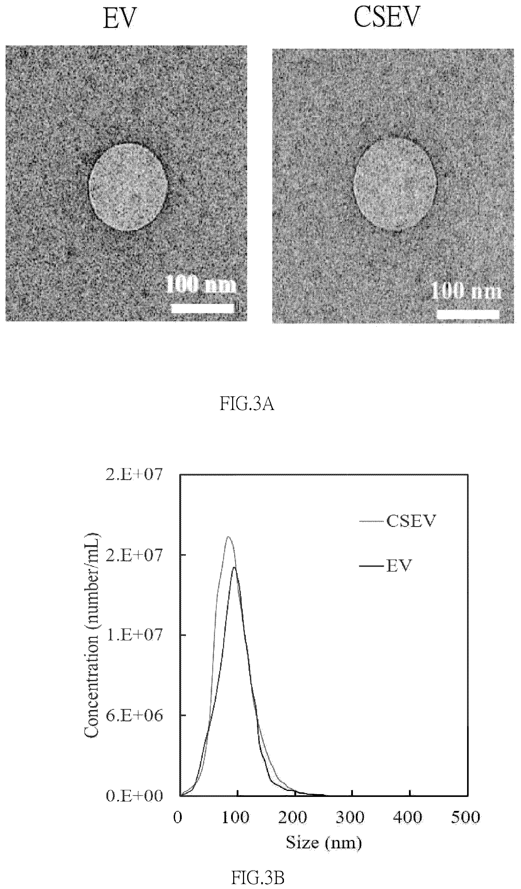

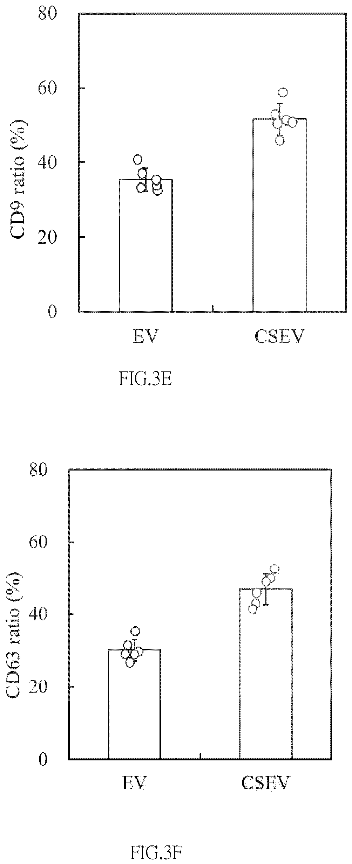

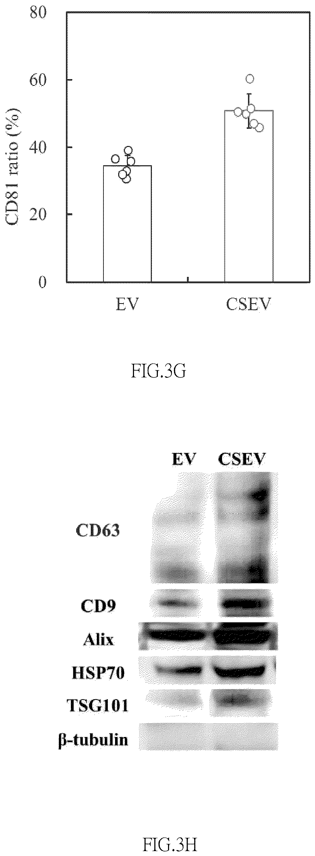

As observed by a transmission electron microscope (TEM) in FIG. 3A, there is little difference in particle size between the ordinary extracellular vesicles (EVs) and the silicate-induced extracellular vesicles (CSEVs), confirming their similar characteristics.

In FIG. 3B, Nanoparticle Tracking Analysis (NTA) not only confirms whether the particle sizes of ordinary extracellular vesicles (EVs) and silicate-induced extracellular vesicles (CSEVs) are comparable, but also detects whether their concentrations are similar. This proves that both ordinary extracellular vesicles (EVs) and silicate-induced extracellular vesicles (CSEVs) belong to the category of the extracellular vesicles.

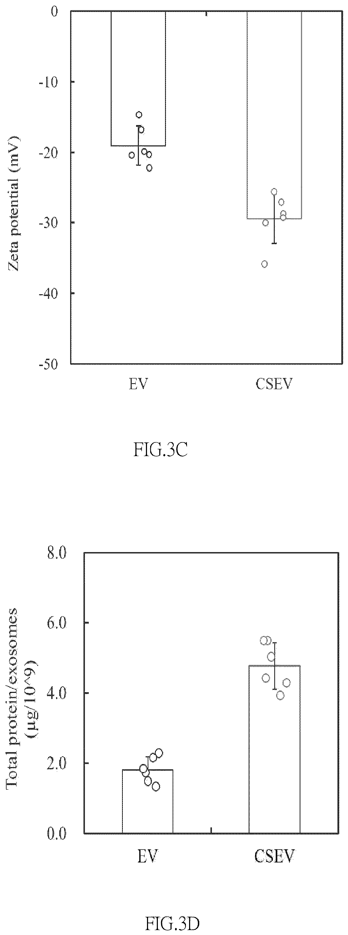

In FIG. 3C, the membrane surface potential of ordinary extracellular vesicles (EVs) and silicate-induced extracellular vesicles (CSEVs) of the present invention is measured. The comparison of the results shows that the membrane surface potential of the silicate-induced extracellular vesicle (CSEV) is always lower than -15 mV, preferably between -20 mV and -30 mV, which is much more negative than that of the ordinary extracellular vesicle (EV).

In FIGS. 3D to 3H, the present invention further analyzes the total protein content of the silicate-induced extracellular vesicles (CSEV) and the expression levels of multiple extracellular vesicle marker proteins using a Western blot method. These marker proteins include transmembrane proteins of CD9, CD63, and CD81, and intramembrane proteins of Alix, TSG101, and HSP70.

The results show that the total protein content of the silicate-induced extracellular vesicle (CSEV) is more than 4µg/10⁹, significantly higher than that of the ordinary extracellular vesicle (EV).

Notably, in over 40% of the silicate-induced extracellular vesicle (CSEV), the transmembrane proteins CD9, CD63, and CD81 are expressed, which play important roles in specific binding, endocytosis, and drug carrying (as shown in FIGS. 3E to 3G).

The above test results indicate that the silicate-induced extracellular vesicle (CSEV) secreted by the human adipose-derived stem cells (ADSCs) after induction with the silicate culture medium show significantly increased surface potential, total protein content, and transmembrane protein content, which helps to increase the affinity of the silicate-induced extracellular vesicle (CSEV) for endocytosis by recipient cells.

To confirm whether the silicate-induced extracellular vesicle (CSEV) has effects on cell proliferation, recovery, and anti-inflammation, the present invention further uses a diabetic cell model commonly used in wound healing, cell proliferation, and recovery research as a test subject.

The establishment of the diabetic cell model refers to an experimental procedure method described in the literature, inducing a human dermal fibroblast (HDF) to simulate the cellular physiological characteristics of a diabetic patient by culturing it in a high glucose culture medium (High Glucose DMEM, HDMEM). In the high glucose culture medium, the glucose concentration is between 13.5 mM and 25 mM.

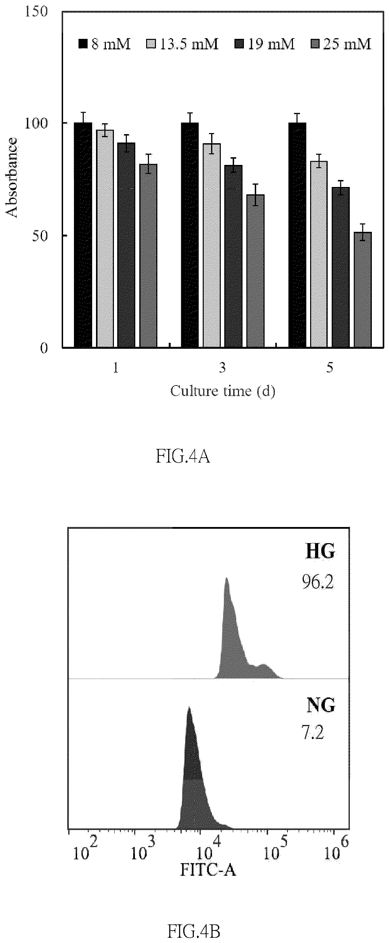

As shown in FIGS. 4A and 4B, the proliferative effect of the human dermal fibroblast decreases with increasing glucose concentration and days in the high glucose culture medium. FIG. 4B compares an intracellular reactive oxygen species (ROS) concentration, which is used as an indicator of inflammatory response, between the human dermal fibroblast group cultured in a high glucose medium with 25 mM glucose (HG) and the group cultured in a normal medium (NG). It can be seen that the expression of reactive oxygen species is significantly higher in the HG group than in the NG group. The decrease in cell proliferation effect and the increase in intracellular reactive oxygen species (ROS) expression can be considered as the main physiological indicators of the diabetic cell model. In the present invention, the diabetic cell model cultured in the high glucose medium with 25 mM glucose concentration will be used for the following experimental explanations.

Experiment 1: Affinity test between the diabetic cell model and the silicate-induced extracellular vesicles. The diabetic cell model was treated with the ordinary extracellular vesicles and the silicate-induced extracellular vesicles separately, and the uptake effect of the diabetic cell model on the ordinary extracellular vesicles or the silicate-induced extracellular vesicles was analyzed after 24 hours and 48 hours of reaction. The group treated with the ordinary extracellular vesicles serves as a control group (EV), while the group treated with the silicate-induced extracellular vesicles serves as an experimental group (CSEV).

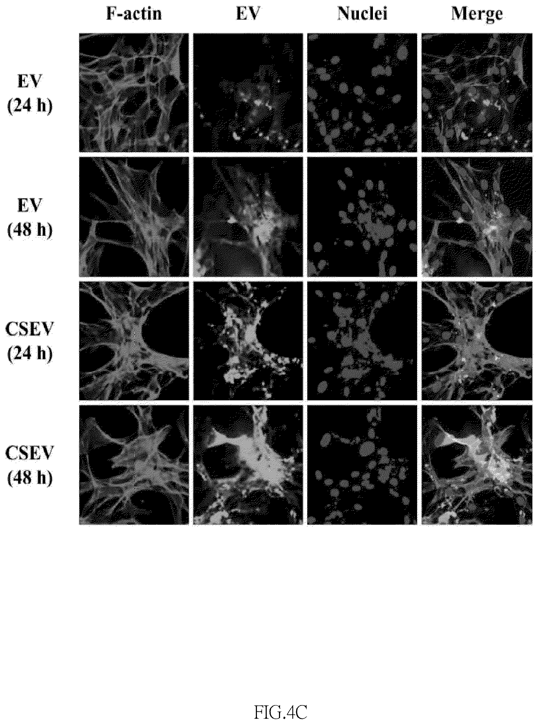

In FIG. 4C, the intracellular range of the diabetic cell model can be defined by a red fluorescence-labeled actin (F-actin), while the ordinary extracellular vesicles and/or the silicate-induced extracellular vesicles are labeled with a green fluorescence, and a blue fluorescence is used to label a cell nucleus. The results show that the green fluorescence labeling the ordinary extracellular vesicles and/or the silicate-induced extracellular vesicles is distributed within the intracellular range defined by the red fluorescence, confirming that the ordinary extracellular vesicles (EVs) and/or the silicate-induced extracellular vesicles (CSEVs) have indeed entered the diabetic cell model through endocytosis.

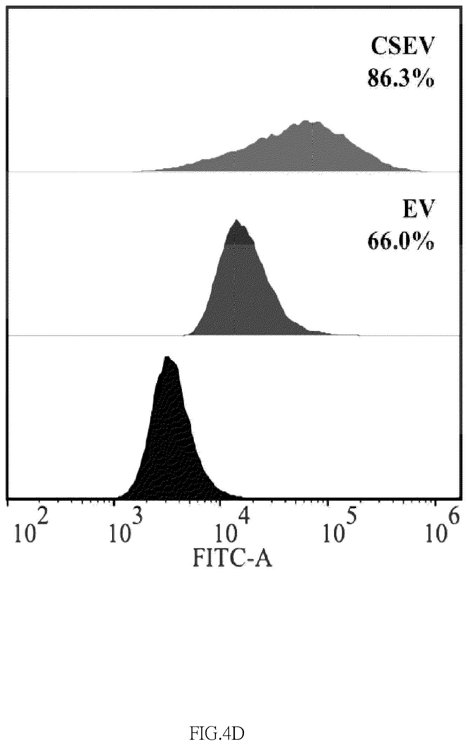

Further observation of the experimental group after 24 hours of reaction with the silicate-induced extracellular vesicles shows that the fluorescence expression of the silicate-induced extracellular vesicles in the diabetic cell model is significantly higher than that in the control group. At 48 hours, the difference in green fluorescence expression between the control group (EV) and the experimental group (CSEV) becomes even more apparent. By detecting the fluorescence intensity of green fluorescence, the data are presented numerically in FIG. 4D, which clearly compares the difference in content between the silicate-induced extracellular vesicles and the ordinary extracellular vesicles in the experimental group (CSEV) and the control group (EV) at 48 hours.

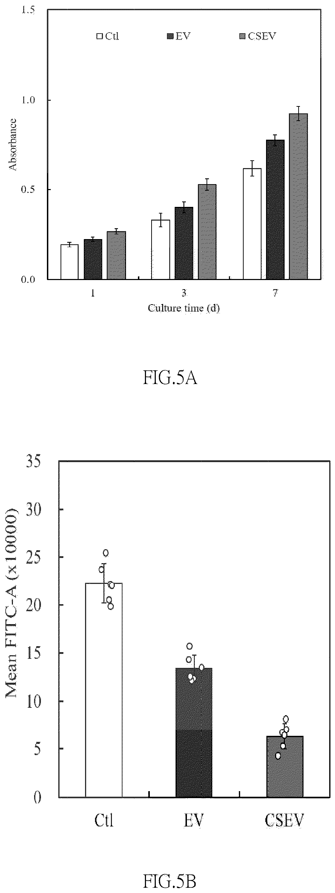

Further confirmation of the cell proliferation effect of the diabetic cell model after co-culture with the silicate-induced extracellular vesicles. In FIG. 5A, through a cell viability test, it can be seen that from the third day onwards, the number of diabetic model cells co-cultured with the silicate-induced extracellular vesicles (experimental group, CSEV) is significantly higher than that of the first control group (Ctl) without any treatment and the second control group (EV) co-cultured with the ordinary extracellular vesicles. In particular, there is a significant difference is produced after 7 days of co-culture. This indicates that the diabetic cell model produces a cell proliferation effect after co-culture treatment with the silicate-induced extracellular vesicles (CSEVs).

FIG. 5B shows the intracellular reactive oxygen species (ROS) concentration measurement of the first control group (Ctl), the second control group (EV), and the experimental group (CSEV) as an indicator of the inflammatory response. The results show that the experimental group (CSEV) of the diabetic cell model after co-culture treatment with the silicate-induced extracellular vesicles has significantly lower intracellular reactive oxygen species expression than the first control group (Ctl) and the second control group (EV).

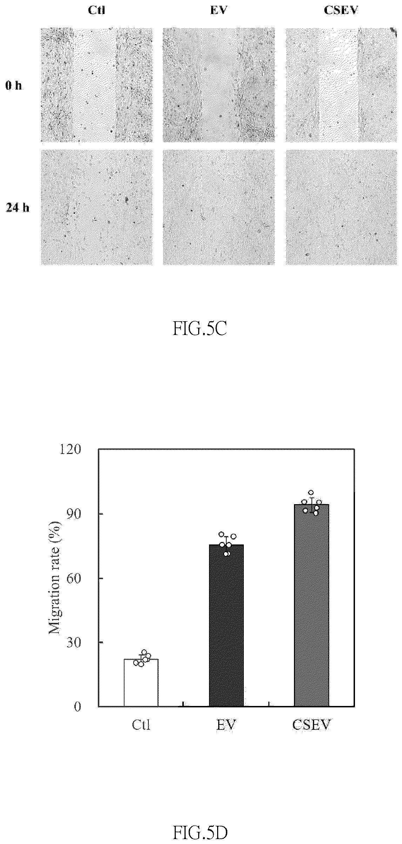

FIG. 5C shows the wound healing assay results for the first control group (Ctl), the second control group (EV), and the experimental group (CSEV). A scratch was made on the cell monolayer of each group, and cell images were captured by microscope at 0 and 24 hours to confirm cell migration effects as an indicator of wound healing. The results show that in the experimental group (CSEV), cells are evenly distributed in the scratch area at 24 hours. When the data are converted into a cell migration percentage based on the proportion of cell coverage in the scratch area (FIG. 5D), it's clear that the cell migration effect of the diabetic cell model after co-culture treatment with silicate-induced extracellular vesicles is significantly superior to both the first control group (Ctl) and the second control group (EV).

These experimental results preliminarily confirm that the silicate-induced extracellular vesicles can promote cell proliferation, cell repair, and reduce the production of anti-inflammatory factors.

Furthermore, the present invention also provides a preparation containing the silicate-induced extracellular vesicle (CSEV), using a hydrogel as a carrier to carry the CSEVs, allowing the preparation to achieve a sustained release effect of the CSEVs.

The hydrogel can be a three-dimensional network structure formed by crosslinking materials such as collagen, gelatin, hyaluronic acid, or chitosan.

The crosslinking can be either physical or chemical. The physical crosslinking can be achieved by principles such as van der Waals forces, ionic attraction, or polymer chain entanglement, while the chemical crosslinking uses covalent bonding principles. In this embodiment, the hydrogel made from a collagen is used for the following embodiment explanations.

The method of carrying the CSEVs on the hydrogel is not limited. In this embodiment, the 5 × 10⁹ CSEVs are mixed with 1 mL of sterile hydrogel to form a mixture. 50 µL of this mixture is extracted into a culture dish and allowed to gel at 37°C for 30 minutes.

The gelation reaction of the mixture can also be achieved through a photocuring technology by adding photosensitizers.

Experiment 2: Comparison of the release effects of ordinary extracellular vesicles and CSEVs carried on the hydrogel. After the gelation reaction with the hydrogel, the ordinary extracellular vesicles and the CSEVs were added to the PBS culture. Solution samples were taken from the culture dish at 0, 1, 2, 3, 5, 7, 10, and 14 hours, and the number of extracellular vesicles in the solution was measured by NTA to calculate the release amount. This confirms that the release rate of CSEVs from the hydrogel is not affected by silicate induction.

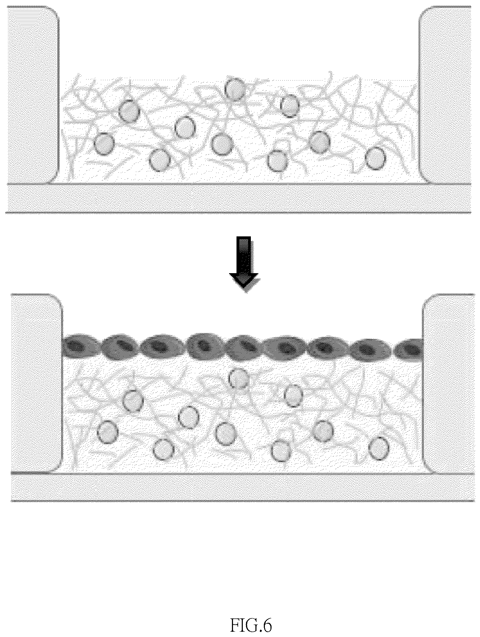

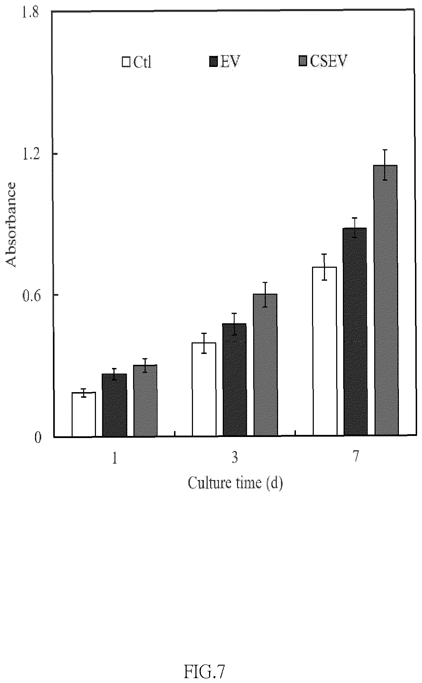

Experiment 3: Comparison of the effects of ordinary extracellular vesicles and CSEVs carried on the hydrogel on the growth of the diabetic cell model. Referring to FIGS. 6 and 7, the hydrogel carrying the ordinary extracellular vesicles and the CSEVs was solidified in the culture dish. Then, 10,000 cells/well of the diabetic cell model were cultured on top of the solidified hydrogel carrying the ordinary extracellular vesicles and/or the CSEV, and the serum-free DMEM was added for culture. The cell proliferation rate was analyzed on days 1, 3, and 7. The hydrogel alone group served as the first control group (Ctl), the hydrogel carried with the ordinary extracellular vesicles as the second control group (EV), and the hydrogel carried with the CSEVs as the experimental group (CSEV).

The results in FIG. 7 show that from the third day, the number of diabetic model cells cultured on the hydrogel with the CSEVs is higher than both the first control group (Ctl) and the second control group (EV). There is a significant difference after 7 days of culture, and this result is consistent with the results in FIG. 5A of Experiment 1, confirming that the CSEV can induce cell proliferation effect in the diabetic cell model.

Experiment 4: Comparison of the effects of ordinary extracellular vesicles and hydrogel-carried CSEVs on the expression of various indicator factors in the diabetic cell model. These indicator factors include inflammation-related factors of TNF-alpha, IL-1, and IL-6; tissue regeneration-related factors of Col I, angiogenin, EGF, FGF-2, and PDGF-BB; anti-inflammatory factors of IL-8 and IL-10; and collagen factor (Col I) as a wound healing indicator.

The diabetic model cells were cultured on the hydrogel alone as the first control group (Ctl), on the hydrogel with ordinary extracellular vesicles as the second control group (EV), and on the hydrogel with the CSEVs as the experimental group (CSEV). The cells were collected, the proteins were extracted and quantified, and 20 µg of protein from each group was analyzed using a cytokine array technology. The expression levels of indicator factors in the second control group (EV) and the experimental group (CSEV) were analyzed based on the expression levels in the first control group (Ctl).

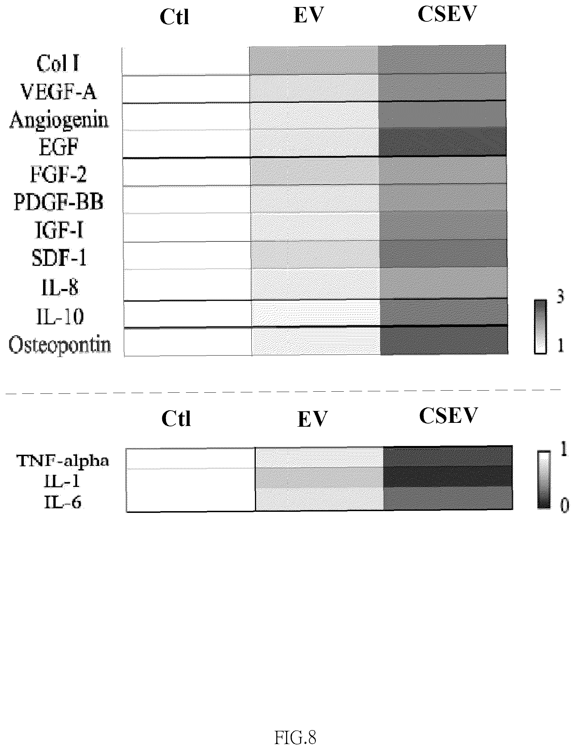

FIG. 8, the results show that compared with the second control group (EV), the diabetic cell model cultured with the experimental group (CSEV) showed significantly decreased expression of inflammation-related factors of TNF-alpha, IL-1, and IL-6; greatly increased expression of tissue regeneration-related factors Col I, angiogenin, EGF, FGF-2, and PDGF-BB; increased expression of anti-inflammatory factors of IL-8 and IL-10; and increased expression of the wound healing-related collagen factor (Col I). These results indicate that the CSEV can induce the expression of anti-inflammatory, tissue regeneration, and cell repair indicator factors in the diabetic model cells.

Further RNA sequencing was performed to compare the expression of microRNAs (miRNAs) in the diabetic model cells cultured with the first control group (Ctl) and the experimental group (CSEV). Small fragment RNA was isolated using an RNAiso (TaKaRa, Japan), and the following sequence analysis was completed by Shanghai OE Biotech Co., Ltd. The miR-Base v.21 database (http://www.mirbase.org/) was used to identify the miRNAs related to angiogenesis, anti-inflammation, and wound healing. The significantly and differentially expressed miRNAs (p < 0.05) were calculated using the differential expression analysis (DEG) algorithm in R language, with three independent replicates for each group. A miRanda software was then used to predict the targets of significantly expressed miRNAs under the parameters of S ≥ 150, ΔG ≤ -30 kcal/mol, and 5' seed pairing in animals, followed by a Gene Ontology (GO) and a Kyoto Encyclopedia of Genes and Genomes (KEGG) methods to analyze the gene locations of different miRNA targets.

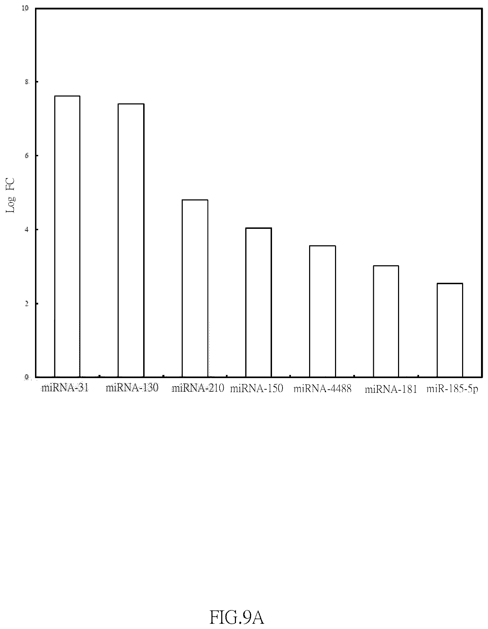

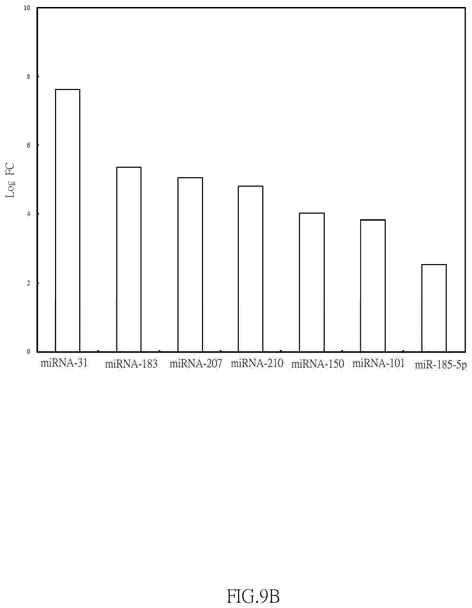

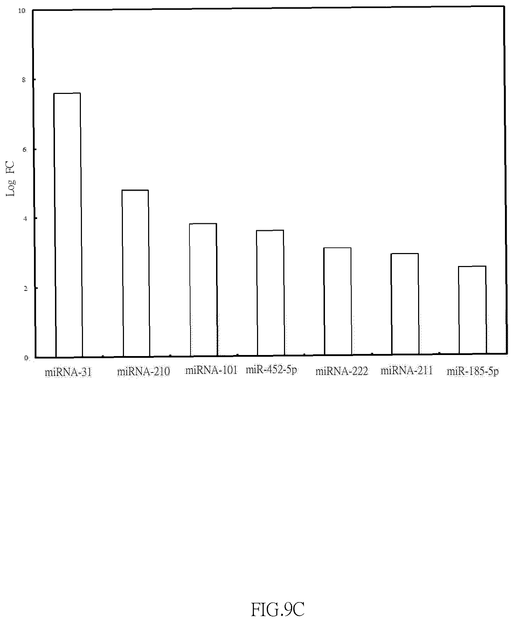

By analyzing the log2 fold change of the data, the ratio between the standardized signal values was obtained to compare the expression of the various miRNAs in diabetic model cells cultured with the experimental group (CSEV) relative to the first control group (Ctl), as shown in FIGS. 9A to 9C. Notably, the diabetic model cells cultured with the experimental group (CSEV) showed significant expression of the indicator miRNA-31 during angiogenesis, anti-inflammation, and wound healing, confirming that the CSEV can induce the expression of anti-inflammatory, tissue regeneration, and cell repair indicator genes (miRNAs) in diabetic model cells.

To confirm the effects of the CSEV on cell proliferation, recovery, and anti-inflammation in real wounds, the present invention further uses a diabetic animal model commonly used in wound healing, cell proliferation, and recovery research as a test subject.

The establishment of the diabetic animal model refers to an experimental procedure method described in the literature in which the chemical agent alloxan monohydrate is used to induce diabetes in the experimental animal. The alloxan induces diabetes by selectively destroying insulin-secreting beta cells in the pancreas, resulting in reduced insulin levels. The alloxan monohydrate used (≥ 98%, Sigma Aldrich Chemical, Saint Louis, MO, USA) is in powder form. Because it easily deteriorates when exposed to air, it is stored sealed in a refrigerator at 2 to 8 degrees Celsius. The drug is prepared by mixing 5.26 grams of the drug with 100 ml of the sterile saline to obtain a 5% reagent (pH=7), which is filtered through a 0.22 µm membrane (all processes are performed on ice), used immediately, and administered by intravenous injection (dose: 100 mg/kg). Blood glucose is measured every two days with the goal of maintaining blood glucose above 300 mg/dl throughout the experiment. If insufficient, alloxan is reintroduced after one week.

Experiment 5: Comparison of the effects of the CSEV on wound repair in the diabetic animal model. After successful establishment of the diabetic animal model, a 1.5 cm² wound is created on the epidermis, with the wound depth reaching the muscle layer, serving as a malignant wound model.

The untreated group serves as the first control group (Ctl), the group using only hydrogel as a wound dressing serves as the second control group (Col), the group using hydrogel with the ordinary extracellular vesicles as the wound dressing serves as the third control group (Ev@Col), and the group using hydrogel with the CSEVs as the wound dressing serves as the experimental group (CSEV@Col). At the second and third weeks after the start of treatment, wound tissues are taken along the wound margins and fixed in 10% formalin. The tissues are then embedded in paraffin and sectioned at 6 µm for a hematoxylin-eosin (H&E) staining and a picrosirius red (PRS) staining. The H&E staining allows observation and quantitative analysis of the thickness of wound epithelial tissue, the amount of granulation tissue, and the formation of mature skin structures (such as hair follicles, sebaceous glands). The PRS allows comparison of the amount and arrangement pattern of collagen fibers at the wound site, thus assessing the repair status of the wound tissue.

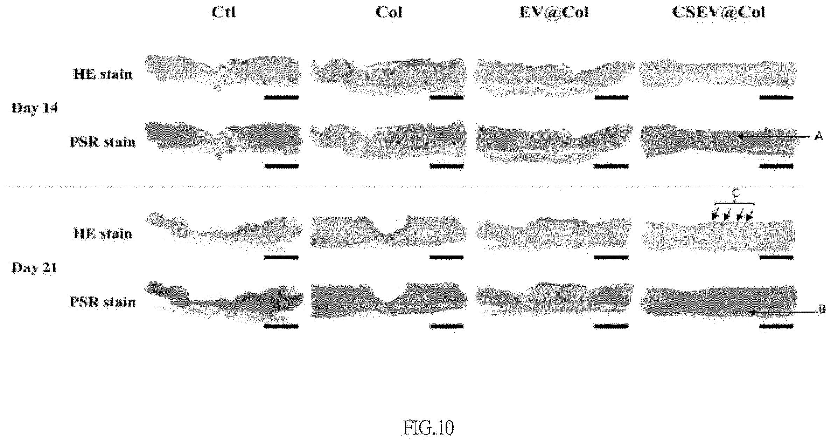

As shown in FIG. 10, the experimental group (CSEV@Col) shows a complete and flat wound appearance compared to the other groups from the second week. In the PRS staining, red-colored, evenly distributed collagen fibers secreted by proliferating dermal fibroblasts can be observed filling the wound in the dermal layer (as indicated by arrow A).

By the third week, the PRS staining shows complete muscle texture in the muscle layer (as indicated by arrow B), and the H&E staining clearly shows the formation of hair follicles in an orderly arrangement in the repaired wound tissue (as indicated by arrow C). This proves that the CSEV promotes cell growth and wound repair in the wound tissue of the diabetic animal model.

In summary, the above experimental results prove that the silicate-induced extracellular vesicles provided by the present invention have high biological safety and can promote in wound repair, thereby achieving the effects of promoting wound healing and repair.

### List of Reference Signs:

Arrows A-C

## Claims

1. A cell repair-promoting extracellular vesicle, formed by silicate-induced secretion of stem cells.

2. The cell repair-promoting extracellular vesicle according to Claim 1, wherein the stem cells are cultured in a silicate culture medium containing a silicate component.

3. The cell repair-promoting extracellular vesicle according to Claim 1, the stem cell comprises a human adipose-derived stem cell (ADSC), a bone marrow-derived stem cell (BMSC), a dental pulp stem cell (DPSC), or a Wharton's jelly MSC (WJMSC).

4. The cell repair-promoting extracellular vesicle according to Claim 1, wherein the silicate is a calcium silicate.

5. The cell repair-promoting extracellular vesicle according to Claim 1, wherein the silicate culture medium comprises a culture medium, which comprises a mesenchymal stem cell medium (MSCM) or a Dulbecco's modified Eagle's medium (DMEM).

6. The cell repair-promoting extracellular vesicle according to Claim 1, comprising a membrane surface potential lower than -15 mV.

7. The cell repair-promoting extracellular vesicle according to Claim 6, wherein the membrane surface potential is between -20 mV and -30 mV.

8. The cell repair-promoting extracellular vesicle according to Claim 1, comprising a protein having a total content of more than 4 µg/10⁹.

9. The cell repair-promoting extracellular vesicle according to Claim 1, wherein at least 40% of the extracellular vesicles express at least one of transmembrane proteins of CD9, CD63, or CD81.

10. A method for producing cell repair-promoting extracellular vesicles formed by silicate-induced stem cell secretion, comprising the steps of:
adding 1 g of calcium silicate to 10 to 100 ml of a culture medium, soaking for at least 24 hours, then collecting the clarified solution to prepare a silicate culture medium;
culturing stem cells in the silicate culture medium; and
isolating extracellular vesicles secreted by the stem cells.

11. The production method according to Claim 10, wherein the culture medium comprises a mesenchymal stem cell medium (MSCM) or a Dulbecco's modified Eagle's medium (DMEM).

12. A cell repair-promoting preparation, comprising an extracellular vesicle formed by silicate-induced stem cell secretion.

13. A cell repair-promoting preparation, comprising a hydrogel carrying an extracellular vesicle formed by silicate-induced stem cell secretion.

14. The cell repair-promoting preparation according to Claim 13, wherein the proportion of the extracellular vesicles (CSEVs) in the hydrogel is between 10⁹-10¹⁴ particles/mL.

15. A cell repair-promoting preparation for use in wound repair, comprising an extracellular vesicle formed by silicate-induced stem cell secretion.

16. The cell repair-promoting preparation for use in wound repair according to Claim 15, wherein the preparation is used for repairing wounds caused by diabetes which are difficult to treat.
